(19) Europäisches Patentamt
European Patent Office
Office européen des brevets

(11) Publication number: **0 024 200**
**B1**

(12) **EUROPEAN PATENT SPECIFICATION**

(45) Date of publication of patent specification: **30.06.82**

(51) Int. Cl.³: **C 07 C 121/16,**
C 07 C 125/08,
C 07 C 149/24,
C 07 D 239/46

(21) Application number: **80302794.5**

(22) Date of filing: **13.08.80**

(54) **N-Cyanoimidates, their preparation and rearrangement to pyrimidines.**

(30) Priority: **14.08.79 US 67354**
**04.10.79 US 81807**

(43) Date of publication of application:
**25.02.81 Bulletin 81/8**

(45) Publication of the grant of the patent:
**30.06.82 Bulletin 82/26**

(84) Designated Contracting States:
**BE DE FR GB IT LU NL**

(56) References cited:
**DE - B - 2 243 317**
**US - A - 3 985 785**
**US - A - 4 032 559**

(73) Proprietor: **E.I. DU PONT DE NEMOURS AND COMPANY**
**Legal Department 1007 Market Street**
**Wilmington Delaware 19898 (US)**

(72) Inventor: **Fuchs, Julius Jakob**
**1104 Greenway Road Forwood**
**Wilmington Delaware 19803 (US)**
Inventor: **Wat, Edward Koon Wah**
**216 West Pembrey Drive**
**Wilmington Delaware 190803 (US)**

(74) Representative: **Skailes, Humphrey John et al,**
**Frank B. Dehn & Co. Imperial House 15-19 Kingsway**
**London WC2B 6UZ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 024 200 B1**

# N-Cyanoimidates, their preparation and rearrangement to pyrimidines

This invention relates to propenimidates, processes for their preparation and processes to prepare pyrimidines therefrom.

*Background of the Invention*

U.S Patent 4,169,719, issued October 2, 1979 describes herbicidal sulfonamides prepared by reacting an appropriate 2-amino pyrimidine with an appropriately substituted sulfonyl isocyanate or isothiocyanate. The described sulfonamides are active herbicides and are especially useful in controlling nutsedge in crops such as cotton, corn, rice and wheat.

The development of an attractive process for preparing the above described sulfonamides by necessity involves inexpensive, easy to carry out processes for preparing the pyrimidine and isocyanate or isothiocyanate intermediates. The pyrimidine intermediate has been difficult to prepare due to hazards associated with raw materials and waste streams. Thus, any simplification in the preparation of the pyrimidine intermediate is desirable.

*Detailed Description of the Invention*

According to the present invention there is provided a N-cyanoimidate of the formula:

I. 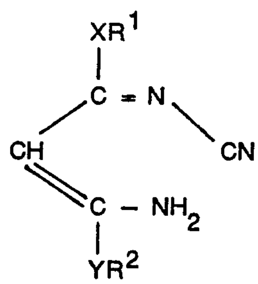

wherein X and Y are independently O or S; and

$R^1$ and $R^2$ are independently $C_1$—$C_4$ alkyl, $(CH_2)_nOR^3$ where $R^3$ is $C_1$—$C_4$ alkyl and n is 1 or 2, $CH_2CH_2Cl$ or $CH_2CF_3$, provided that when $R^1$ and $R^2$ is $CH_2CH_2Cl$ or $CH_2CF_3$, then the respective X or Y is O.

In the compounds of the invention, X and Y are preferably both O or both S, most preferably O; and $R^1$ and $R^2$ are preferably independently methyl or ethyl, most preferably both are methyl. Thus, the preferred compound of the invention is methyl 3-amino-3-methoxy-N-cyano-2-propenimidate.

There is also provided a process for preparing the above-described N-cyanoimidate which is characterized by:

a) contacting a dihydrohalide salt of the formula:

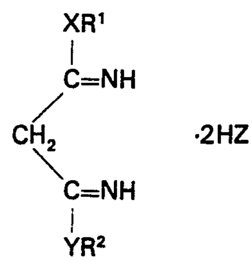

wherein X, Y, $R^1$ and $R^2$ are as defined as above and Z is F, Cl or Br,

(i) in an inert liquid medium with one equivalent weight of a base at a pH no higher than 7, to produce a monohydrohalide salt, or

(ii) with two mole equivalents of a base to produce a bisimidate, and

b) (i) contacting the monohydrohalide salt from (a) (i) with cyanamide, or

(ii) contacting the bisimidate from (a) (ii) with cyanogen chloride to produce a mixture of an N-cyanoimidate and a monohydrohalide bisimidate salt, and isolating the N-cyanoimidate from the reaction mixture.

Further provided is a process for preparing a compound of the formula:

IV. 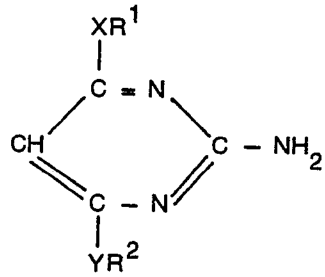

wherein X, Y, $R^1$ and $R^2$ are as defined above, which comprises heating the N-cyanoimidate according to the present invention at a temperature sufficient to ring close.

The process of the invention for preparing compounds of the invention (I) and their subsequent conversion to pyrimidines (IV) useful as herbicide intermediates is exemplified by the following reactions:

PROCESS 1

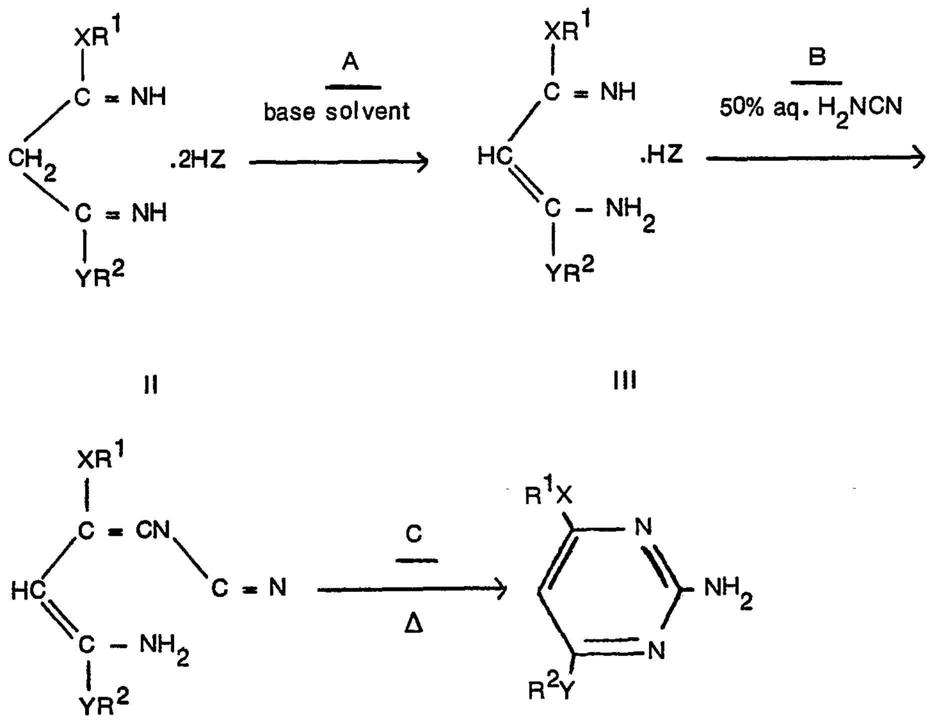


II III

I IV

PROCESS 2

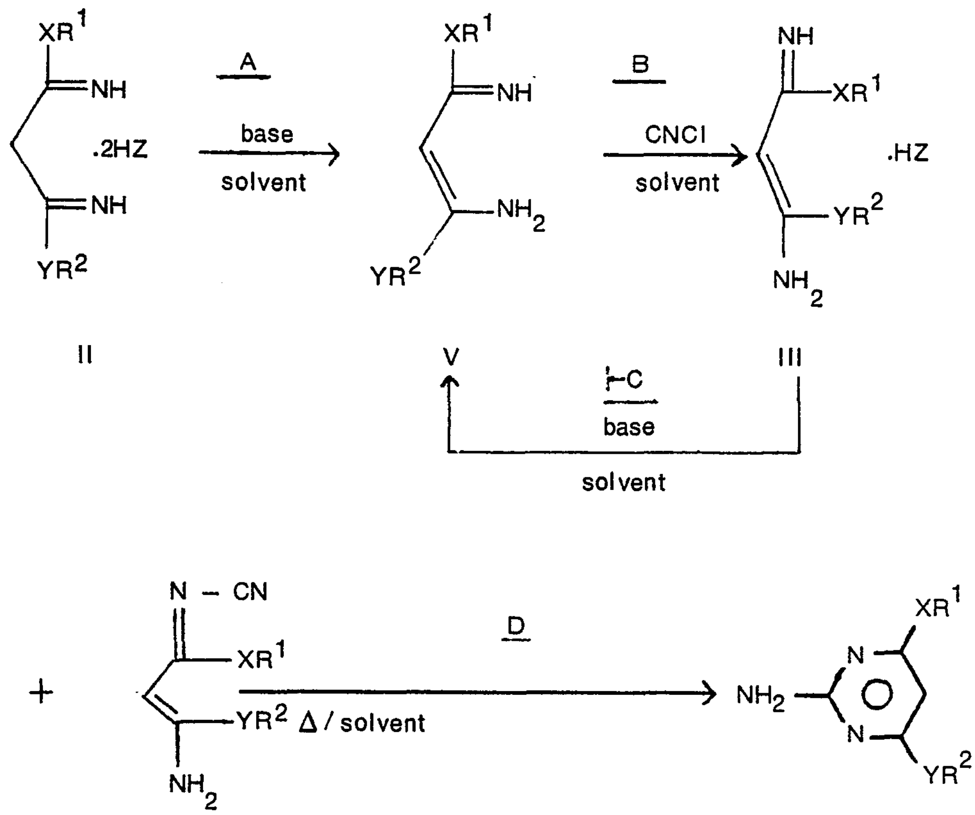


II V III I IV

In the above reactions Z is F, Cl, or Br and X, Y, $R^1$ and $R^2$ are as defined previously.

In the first step of process 1, reaction *A*, a dihydrohalide salt, preferably a dihydrochloride salt, of a bismalonimidate of formula II is contacted with one equivalent weight of a base at a pH no higher than 7 in an inert liquid medium to produce a monohydrohalide salt (III). The base is preferably an alkali metal (preferably sodium) bicarbonate, carbonate, hydroxide or methoxide, pyridine or triethylamine (or any other tertiary amine base). Sodium carbonate, sodium bicarbonate or sodium hydroxide are the most preferred bases usually used as their aqueous solutions. The liquid medium used to suspend or dissolve the reactants can be any liquid inert to the reaction. Typical liquids are halogenated compounds such as $CH_2CL_2$, or $CHCl_1$, ethers such as diethylether or dioxane, esters such as methylacetate or ethylacetate, alcohols such as methanol or ethanol, or hydrocarbons such as benzene, toluene, hexane or cyclohexane. The pH of the reaction mixture is preferably maintained in the range of 5—7. Temperature of the reaction is preferably in the range of −10°C to 20°C.

Reaction *A* of process 1 can preferably be carried out by any one of the following procedures:

1) to a suspension of (II) in a liquid in which the synthesis of (II) has been performed, is added at −10 to 20°C enough base to neutralize any excess free hydrohalide (chloride) acid which may still be present from the synthesis of (II), plus one equivalent of base to convert (II) to (III);

2) solid (II) is suspended in any one of the described liquid mediums and then contacted with one equivalent of base at −10 to 20°C;

3) solid (II) is added to one equivalent of one of the preferred aqueous bases, i.e., $Na_2CO_3$, $NaHCO_3$ or NaOH, at −10 to 20°C; or

4) a suspension of (II) in one of the described liquid mediums is added simultaneously with one equivalent of an aqueous base to ice water, maintaining a pH of 5—7 by regulating the addition rate of either stream. The preferred procedure is either 3) or 4).

A compound of (II), dimethyl 1,3-propanediimidate dihydrochloride (X and Y=0; $R^1$ and $R^2$=methyl), is known in the art. S. M. McElvain and J. P. Schroeder, JACS *71*, 40 (1949); B. Harsteen, German Offenlegungsschrift 2,426,913, Dec. 11, 1975. Other compounds of (II) can be made by the procedures described.

In reaction *B*, of Process 1, the monohydrohalide salt compound of formula (III) is contacted with cyanamide ($H_2NCN$), preferably 50 percent by weight aqueous cyanamide. The reaction is preferably carried out in a solvent such as water, a lower alcohol or mixtures, thereof, preferably water. The reaction can also be carried out in the presence of a water-immiscible solvent such as methylene

chloride to extract compound (I) as it is formed. Temperature of reaction is usually in the range of 20—75°C, preferably 25—50°C, for a time sufficient to give compound (I), i.e., 15 minutes to 5 hours, normally about 1 to 3 hours.

Reaction *B* of Process 1 can be carried out by one of the following procedures:

1) to the reaction mass obtained in reaction *A* 1), 2) or 4) is added an equivalent or a slight excess (about 10%) of cyanamide, preferably as 50% aqueous cyanamide, and the reaction mass stirred until the reaction is completed (up to 5 hours at 20°C to 15 minutes at 75°C). Water is then added to dissolve any salts present. Any undissolved product (I) is then isolated by filtration, then the aqueous phase is separated from the organic phase and (I) isolated from the organic phase by evaporation of the solvent; or

2) to the solution obtained in reaction *A* 3) is added an equivalent or a slight excess (about 10%) of aqueous cyanamide and the solution warmed to room temperature where it is held for about 2 hours. Solid product (I), which precipitates during this time, is then isolated by filtration.

Reaction *A* of Process 2 involves the conversion of dihydrohalide salt II to the bisimidate of Formula V. This is done by contacting, in an inert liquid medium the dihydrohalide salt with two mole equivalents of a base at a pH no lower than about 8. The base is preferably an alkali metal or alkaline earth carbonate or hydroxide and more preferably an alkali metal carbonate such as sodium or potassium carbonate.

Preferably, the salt II, which is suspended in an inert organic liquid medium, is added to an aqueous solution of the base. Suitable liquid mediums include chloroform and methylene chloride, the latter being preferred. The aqueous base to solvent ratio is preferably about 1:1 although higher or lower ratios are operable. The reaction mixture is cooled to maintain a temperature within the range of −10° to +50°C, preferably in the range of −5°C to +5°C. The product bisimidate is contained in the organic phase.

Reaction *A* of Process 2 is exemplified by the following procedure: A suspension of II in one of the described liquid mediums is added to a solution or suspension of at least two equivalents of an aqueous base in ice-water. The organic phase containing the product V is removed and the water layer is further extracted with additional organic solvent. The combined organic solutions are dried over magnesium sulfate or sodium sulfate. If a different solvent is to be used in Reaction *B* of Process 2, evaporation of solvent at reduced pressure yields an oily product.

In Reaction *B* of Process 2, the bisimidate V is contacted with cyanogen chloride to prepare a mixture of the monohydrohalide salt III and the N-cyanoimidate I. The reactants are combined in the organic layer retrieved from Reaction *A* of Process 2, or, if the bisimidate has been isolated, in an inert organic solvent. Suitable solvents include methylene chloride, chloroform, tetrahydrofuran and dioxane, with methylene chloride being preferred. The cyanogen chloride is preferably in the amount of about one-half to about one mole per mole of bisimidate. The reaction is run at a temperature of 0°C to 80°C, preferably 20°C to 45°C. When the solvent used is methylene chloride, pressure must be applied to achieve temperatures above reflux. The reaction time will vary with the temperature selected. For example, at the reflux temperature of methylene chloride, a reaction time of approximately 3 hours is required.

The monohydrohalide salt III precipitates during the reaction. Thus, the desired N-cyanoimidate I may be isolated by filtration followed by evaporation of the solvent.

Reaction *B* of Process 2 is exemplified by the following procedure:

Gas or liquid cyanogen chloride is added to the solution of V prepared in Reaction *A*. Alternatively, solvent-free V is dissolved or suspended in an inert liquid medium to which cyanogen chloride is added. The mixture is heated, and a precipitate of monohydrohalide salt III is formed. The mixture is cooled to 25°C and filtered. The filtrate contains the desired N-cyanoimidate I which is isolated by evaporation of the solvent at reduced pressure. A solid residue remains which is further refined by washing with ether.

The monohydrohalide salt III may be recycled to produce the bisimidate V by following the procedure of Reaction *A*. Unlike Reaction *A*, however, only one equivalent of base is needed to neutralize the salt.

As shown in reaction *C* of Process 1 or reaction *D* of Process 2 compounds of the invention (I) can be caused to undergo ring closure to the herbicide intermediates (IV) by heating. Preferably, compound (I) is dissolved or suspended in a suitable inert liquid medium such as water, methanol, toluene or xylene and maintained at a temperature in the range of 0 to 200°C, preferably about 50—150°C, until the rearrangement is complete. For lower boiling solvents, sufficient pressure is applied to achieve the desired reaction temperature. Alternatively, ring closure can be accomplished by heating the neat compound of formula (I) to a temperature above its melting point for a suitable period of time.

Reactions *C* and *D* are exemplified by one of the following procedures:

1) a solution or suspension of (I) in an inert liquid medium is heated to 65—110°C until rearrangement is complete (several hours at 65°C to about 60 minutes at 110°C) then the solution or suspension is cooled and the pyrimidine (IV) isolated by filtration or evaporation of the solvent;

2) a compound of formula (I) is heated neat to its melting point, e.g., when X and Y = O and $R^1$ and $R^2$ = methyl, heating to about 130°C will give ring closure in less than one minute.

The ring closure reaction of either procedure is highly exothermic and can lead to boiling of the

liquid or a considerable rise in temperature of the melt when (I) is heated neat. Reactions *C* and *D* 1) can easily be carried out from the reaction mass obtained from reaction *B* of Process 1, without isolation of product (I), by heating and distilling out any organic solvent present, either as neat solvent or as an azeotrope with water. In the latter case, the aqueous solution of (I) is maintained at temperature to complete the ring closure. After cooling, (IV) is isolated by filtration.

The pyrimidine of formula (IV) is reacted with a substituted sulfonyl isocyanate or isothiocyanate as described in the aforesaid U.S. Patent 4,169,719 to produce a herbicidal sulfonamide. This patent is hereby incorporated by reference.

By using the procedures described, the following compounds of formula (I) can be prepared:

| X | Y | $R^1$ | $R^2$ |
|---|---|---|---|
| O | O | $CH_3$ | $CH_3$ |
| O | O | $CH_3$ | $CH_2CH_2CH_2CH_3$ |
| O | O | $CH_3$ | $CHCH_3$<br>\|<br>$CH_3$ |
| O | O | $CH_3$ | $CH_2CH_2OCH_3$ |
| O | O | $CH_3$ | $CH_2OCH_2CH_2CH_2CH_3$ |
| O | O | $CH_3$ | $CH_2CH_2Cl$ |
| O | O | $CH_3$ | $CH_2CF_3$ |
| O | S | $CH_3$ | $CH_3$ |
| O | S | $CH_3$ | $—CHCH_2CH_3$<br>\|<br>$CH_3$ |
| S | S | $CH_3$ | $CH_3$ |
| S | S | $CH_3$ | $CH_2CH_3$ |
| S | S | $CH_3$ | $—CH_2CHCH_3$<br>\|<br>$CH_3$ |
| S | S | $CH_3$ | $CH_2OCH—CH_3$<br>\|<br>$CH_3$ |
| S | S | $CH_3$ | $CH_2CH_2OCH_3$ |
| O | O | $CHCH_3$<br>\|<br>$CH_3$ | $CHCH_3$<br>\|<br>$CH_3$ |
| O | O | $CH_2CH_3$ | $CH_2CH_3$ |
| S | S | $CH_2CH_3$ | $CH_2CH_3$ |
| O | S | $CH_2CH_3$ | $CH_2CH_3$ |
| O | O | $CH_3$ | $CH_2CH_3$ |
| O | O | $CH_3$ | $—CH_2CHCH_3$<br>\|<br>$CH_3$ |

| X | Y | $R^1$ | $R^2$ |
|---|---|---|---|
| O | O | $CH_3$ | $—CHCH_2CH_3$<br>$CH_3$ |
| O | O | $CH_3$ | $CH_2OCHCH_3$<br>$CH_3$ |
| O | O | $CH_3$ | $CH_2OCH_2CH_3$ |
| O | O | $CH_3$ | $CH_2OCHCH_2CH_3$<br>$CH_3$ |
| O | O | $CH_3$ | $CH_2CH_2OCH_2CH_3$ |
| O | O | $CH_3$ | $CH_2CH_2OCHCH_3$<br>$CH_3$ |

The invention can be further understood by the following examples in which parts and percentages are by weight unless otherwise indicated.

Example 1

a) A suspension of 101.5 g of dimethyl 1,3-propanediimidate dihydrochloride in 200 ml of methanol was treated at 0°C with 216 g of a 25% solution of sodium methylate in methanol until a pH of 6 was obtained. Solid NaCl, which had been precipitated, was then removed by filtration and the filtrate evaporated under vacuum until a viscous solution of the monohydrochloride salt in methanol remained. This solution was then triturated with acetone to precipitate the monohydrochloride salt, which was recovered by filtration. Melting point 91—93°C; 21.3% chloride content; yield 90%.

b) The monohydrochloride salt from above (16.7 g) was heated with 10 g of 50% aqueous cyanamide, 10 ml of water and 300 ml of $CH_2Cl_2$ for one hour at 40°C. The organic phase was then separated, dried and the solvent removed under vacuum to give 12.8 g of methyl 3-amino-3-methoxy-N-cyano-2-propenimidate mp 128—130°C.

Example 2

To a suspension of 42 g $NaHCO_3$, in 400 ml of water at 0°C, was added, in small portions, at a pH greater than 5, 101.5 g of dimethyl 1,3-propanediimidate dihydrochloride. To the resulting solution was added 45 parts of 50% aqueous cyanamide and the solution warmed to room temperature where it was held for 2 hours. Solid product, methyl 3-amino-3-methoxy-N-cyano-2-propenimidate, precipitated during this time, which was then removed by filtration, washed with water and dried, mp 128—129°C, after recrystallization from methanol, mp 131—132°C. A small second crop of product was isolated from the aqueous filtrate by extraction with $CH_2Cl_2$ and evaporation of solvent.

Example 3

One gram of recrystallized product from Example 2 was heated in a test tube with the aid of an oil bath to its melting point of 131°C, at which point a sudden release of energy raised the temperature of the melt to 190°C. The melt was then cooled and solidified. The resulting product melted at 94—96°C and its IR spectrum was identical with pure 2-amino-4,6-dimethoxypyrimidine.

Example 4

A suspension of 5 g of recrystallized product from Example 2 in toluene was refluxed for 60 minutes. The toluene was evaporated from the resulting solution to give 2-amino-4,6-dimethoxy-pyrimidine in quantitative yield, mp 94—96°C.

Example 5

A suspension of 101.5 g of dimethyl 1,3-propanediimidate dihydrochloride in 2 liters of $CH_2Cl_2$ was gradually added together with 50% NaOH to 400 ml of water contained in a reactor, maintained at 0 to −5°C, at rates such that the pH of the aqueous solution was maintained between 5—7. Then, 45 g of 50% aqueous cyanamide was added and the resulting two-phase reaction mass refluxed at 40°C for one hour. After phase separation, the $CH_2Cl_2$ phase was dried by distilling $CH_2Cl_2$ and azeotroping the water. The resulting solution of methyl 3-amino-3-methoxy-N-cyano-2-propenimidate in $CH_2Cl_2$ was gradually fed to xylene maintaining the temperature at 100—130°C while flashing off and

condensing $CH_2Cl_2$. After all of the solution was fed, the solution was held at temperature for 15 minutes and then filtered. There was obtained 2-amino-4,6-dimethoxypyrimidine, mp 94—96°C.

Example 6

Dimethyl-1,3-propendiimidate

To a stirred mixture of 400 ml aqueous potassium carbonate (300 g/liter) and 400 ml methylene chloride, 79 g of 1,3-propanediimidate dihydrochloride was added in portions. The organic layer was removed, and the aqueous layer was extracted with three-80 ml portion of methylene chloride. The combined organic layers were dried over anhydrous sodium sulfate and then filtered. The filtrate was evaporated to yield 51 g of a colorless liquid. This crude product was used in the next example without further purification.

Example 7

Methyl 3-Amino-3-methoxy-N-cyano-2-propenimidate

a) A 3.5 ml portion of condensed cyanogen chloride was added to a solution of 8.4 g of the product of Example 6 in 100 ml tetrahydrofuran. The solution was stirred at 25° and a white solid began to form after ten minutes. After being stirred for 2 hours, the mixture was filtered. The solids were washed with tetrahydrofuran and dried to yield 4.5 g of a white solid, methyl 3-amino-3-methoxy-2-propenimidate hydrochloride, m.p. 72—75°. The filtrate was evaporated at reduced pressure to give a solid residue containing a small amount of oil. A small volume of ether was added, and the mixture was filtered to give 3.6 g of methyl 3-amino-3-methoxy-N-cyano-2-propenimidate, m.p. 116—121°. A second crop 0,5 g, was obtained from the ether solution to give an overall yield of 41%. This yield may be increased by recycling the monohydrohalide salt.

b) The procedure of part (a) was repeated using 25.5 g of the product of Example 6 in 150 ml methylene chloride and 10.1 ml cyanogen chloride. The mixture was refluxed for 3 hours. This reaction yielded 13.3 g of methyl 3-amino-3-methoxy-N-cyano-2-propenimidate and 12.2 g of methyl 3-amino-3-methoxy-2-propenimidate hydrochloride.

Example 8

2-Amino-4,6-dimethoxypyrimidine

A mixture of 56.4 g of methyl 3-amino-3-methoxy-N-cyano-2-propenimidate in 200 ml toluene was refluxed for 4 hours. The resulting homogeneous solution was cooled to 0° and a solid precipitated. It was removed by filtration to yield 48.8 g of pale yellow solid, m.p. 92—93.5°. A second crop of 5.2 g was obtained from the mother liquors for a total yield of 96%.

**Claims**

1. An N-cyanoimidate of the formula:

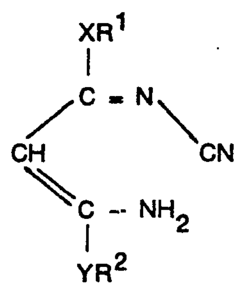

wherein X and Y are independently O or S; and

$R^1$ and $R^2$ are independently $C_1$—$C_4$ alkyl, $(CH_2)_nOR^3$ where $R^3$ is $C_1$—$C_4$ alkyl and n is 1 or 2, $CH_2CH_2Cl$ or $CH_2CF_3$, provided that when $R^1$ or $R^2$ is $CH_2CH_2Cl$ or $CH_2CF_3$, then the respective X or Y is O.

2. A compound of Claim 1 wherein X and Y are both O or both S and $R^1$ and $R^2$ are independently methyl or ethyl.

3. A compound of Claim 1 wherein X and Y are O and $R^1$ and $R^2$ are methyl.

4. A process for preparing an N-cyanoimidate of any of Claims 1—3 characterized by:

a) contacting a dihydrohalide salt of the formula

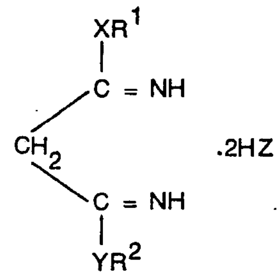

wherein X, Y, $R^1$ and $R^2$ are as defined in Claims 1—3 and Z is F, Cl or Br,

(i) in an inert liquid medium with one equivalent weight of a base at a pH no higher than 7, to produce a monohydrohalide salt, or

(ii) with two mole equivalents of a base to produce a bisimidate, and

b) (i) contacting the monohydrohalide salt from (a) (i) with cyanamide, or

(ii) contacting the bisimidate from (a) (ii) with cyanogen chloride to produce a mixture of an N-cyanoimidate and a monohydrohalide bisimidate salt, and isolating the N-cyanoimidate from the reaction mixture.

5. The process of Claim 4 characterized in that the monohydrohalide in (b) (i) is contacted with a solution of cyanamide in a solvent selected from water, a lower alcohol and a mixture thereof preferably water at a temperature in the range of 20—75°C, preferably 25—50°C or the bisimidate in (b) (ii) as a solution in methylene chloride is contacted with cyanogen chloride at a temperature of 0 to 80°C.

6. The process of Claim 4 or Claim 5 characterized in that the dihydrohalide in (a) (i) is contacted with an aqueous solution of sodium carbonate, sodium bicarbonate or sodium hydroxide at a temperature in the range of −10°C to 20°C and at a pH of the reaction medium in the range of 5—7 or the dihydrohalide salt in (a) (ii) as a suspension in methylene chloride is contacted with an aqueous solution of base at a temperature in the range of −10 to 50°C.

7. The process of any of Claims 4—6 characterized in that the monohydrohalide bisimidate salt formed in (b) (ii) is recycled by

a) contacting the monohydrohalide bisimidate salt with one mole equivalent of a base to produce a bisimidate, and

b) contacting the bisimidate with cyanogen chloride to produce an N-cyanoimidate.

8. The process of any of Claims 4 to 7 characterized in that the resulting N-cyanoimidate is subsequently heated at a temperature sufficient to ring close and form a compound of the formula:

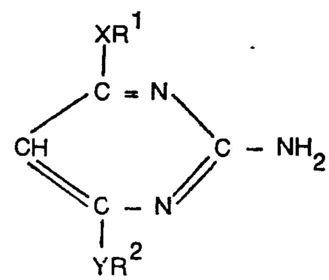

9. The process of Claim 8 characterized in that the N-cyanoimidate is heated (a) neat at a temperature above its melting point or (b) in an inert liquid medium at a temperature in the range of 0 to 200°C, until ring closure is complete.

**Revendications**

1. Un N-cyano-imidate de la formule:

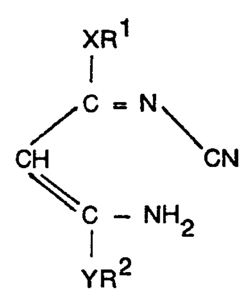

dans laquelle X et Y sont indépendamment O ou S; et

$R^1$ et $R^2$ sont indépendamment des groupes alcoyle en $C_1$—$C_4$, $(CH_2)_nOR^3$ où $R^3$ est un groupe alcoyle en $C_1$—$C_4$ et *n* est 1 ou 2, $CH_2CH_2Cl$ ou $CH_2CF_3$, avec la condition que, quand $R^1$ ou $R^2$ est $CH_2CH_2Cl$ ou $CH_2CF_3$, alors le X ou Y respectif est O.

2. Un composé selon la revendication 1 dans lequel X et Y sont tous deux O ou tous deux S, et $R^1$ et $R^2$ sont indépendamment des groupes méthyle ou éthyle.

3. Un composé selon la revendication 1 dans lequel X et Y sont O et $R^1$ et $R^2$ sont des groupes méthyle.

4. Un procédé pour préparer un N-cyano-imidate selon l'une quelconque des revendications 1 à 3, caractérisé en ce que:

a) on met en contact un sel dihalogénhydrate de la formule:

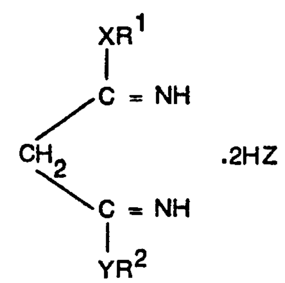

dans laquelle X, Y, $R^1$ et $R^2$ sont tels que définis dans les revendications 1 à 3 et Z est F, Cl ou Br,

(i) dans un milieu liquide inerte avec un poids équivalent d'une base à un pH pas supérieur à 7, afin de produire un sel monohalogénhydrate, ou

(ii) avec deux équivalents molaires d'une base afin de produire un bisimidate, et

b) (i) on met en contact le sel monohalogénhydrate résultant de (a)(i) avec du cyanamide, ou

(ii) on met en contact le bisimidate résultant de (a) (ii) avec du chlorure de cyanogène afin de produire un mélange d'un N-cyano-imidate et d'un sel monohalogénhydrate de bisimidate, et on isole le N-cyano-imidate à partir du mélange de réaction.

5. Le procédé selon la revendication 4, caractérisé en ce que le monohalogénhydrate dans (b) (i) est mis en contact avec une solution de cyanamide dans un solvant choisi parmi l'eau, un alcool inférieur et un mélange de tels solvants, de préférence de l'eau à une température comprise entre 20 et 75°C, en particulier entre 25 et 50°C, ou le bisimidate dans (b) (ii) sous la forme d'une solution dans le chlorure de méthylène est mis en contact avec du chlorure de cyanogène à une température de 0 à 80°C.

6. Le procédé selon la revendication 4 ou la revendication 5, caractérisé en ce que le dihalogènhydrate dans (a) (i) est mis en contact avec une solution aqueuse de carbonate de sodium, de bicarbonate de sodium ou d'hydroxyde de sodium à une température comprise entre −10°C et 20°C et à un pH du milieu de réaction compris entre 5 et 7, ou le sel dihalogénhydrate dans (a) (ii) sous la forme d'une suspension dans du chlorure de méthylène est mis en contact avec une solution aqueuse de base à une température comprise entre −10 et 50°C.

7. Le procédé selon l'une quelconque des revendications 4 à 6, caractérisé en ce que le sel monohalogénhydrate de bisimidate formé dans (b) (ii) est recyclé par

a) mise en contact du sel monohalogénhydrate de bisimidate avec un équivalent molaire d'une base afin de produire un bisimidate, et

b) mise en contact du bisimidate avec du chlorure de cyanogène afin de produire un N-cyano-imidate.

8. Le procédé selon l'une quelconque des revendications 4 à 7, caractérisé en ce que le N-cyano-imidate résultant est ensuite chauffé à une température suffisante pour être cyclisé et former un composé de la formule:

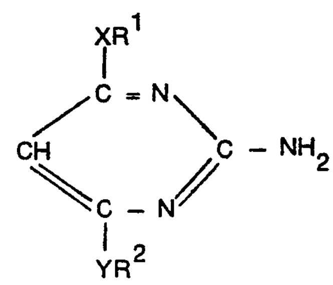

9. Le procédé selon la revendication 8, caractérisé en ce que le N-cyano-imidate est chauffé (a) sans solvant à une température plus élevée que son point de fusion ou (b) dans un milieu liquide inerte à une température comprise entre 0 et 200°C, jusqu'à ce que la cyclisation soit complète.

**Patentansprüche**

1. N-Cyanoimidat der Formel

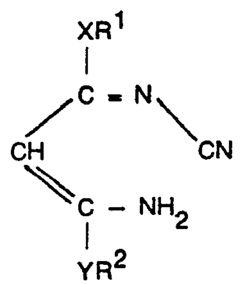

worin X und Y unabhängig O oder S sind; und $R^1$ und $R^2$ unabhängig $C_1$—$C_4$-Alkyl, $(CH_2)_nOR^3$, worin $R^3$ $C_1$—$C_4$-Alkyl ist und n für 1 oder 2 steht, $CH_2CH_2Cl$ oder $CH_2CF_3$ sind, vorausgesetzt, dass, wenn $R^1$ oder $R^2$ die Bedeutung von $CH_2CH_2Cl$ oder $CH_2CF_3$ hat, das jeweilige X oder Y für O steht.

2. Verbindung nach Anspruch 1, worin X und Y beide O oder beide S sind und $R^1$ und $R^2$ unabhängig Methyl oder Äthyl sind.

3. Verbindung nach Anspruch 1, worin X und Y die Bedeutung von O haben und $R^1$ und $R^2$ Methyl sind.

4. Verfahren zur Herstellung eines N-Cyanoimidats nach einem der Ansprüche 1—3, dadurch gekennzeichnet, dass man

a) ein Dihydrohalogenidsalz der Formel

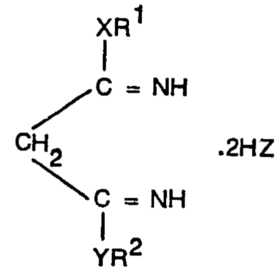

worin X, Y, $R^1$ und $R^2$ wie in den Ansprüchen 1—3 definiert sind un Z für F, Cl oder Br steht,

(i) in einem inerten flüssigen Medium mit einem Äquivalentgewicht einer Base bei einem pH-Wert von nicht höher als 7 unter Bildung eines Monohydrohalogenidsalzes, oder

(ii) mit zwei Mol-Äquivalenten einer Base, unter Bildung eines Bisimidats, in Kontakt bringt, und

b) (i) das Monohydrohalogenidsalz von a) (i) mit Cyanamid in Kontakt bringt, oder

(ii) das Bisimidat von a) (ii) mit Chlorcyan in Kontakt bringt, unter Bildung eines Gemischs eines N-Cyanoimidats und eines Monohydrohalogenid-Bisimidatsalzes, und das N-Cyanoimidat aus dem Reaktionsgemisch isoliert.

5. Verfahren nach Anspruch 4, dadurch gekennzeichnet, dass man das Monohydrohalogenid in b) (i) mit einer Lösung von Cyanamid in einem Lösungsmittel, ausgewählt aus Wasser, einem niedrigen Alkohol und einem Gemisch davon, vorzugsweise Wasser, bei einer Temperatur im Bereich von 20—75°C, vorzugsweise 25—50°C in Kontakt bringt, oder das Bisimidat in b) (ii) als eine Lösung in Methylenchlorid mit Chlorcyan bei einer Temperatur von 0 bis 80°C in Kontakt bringt.

6. Verfahren nach Anspruch 4 oder Anspruch 5, dadurch gekennzeichnet, dass man das Dihydrohalogenid in a) (i) mit einer wässrigen Lösung von Natriumcarbonat, Natriumbicarbonat oder Natriumhydroxid bei einer Temperatur im Bereich von —10°C bis 20°C und bei einem pH-Wert des Reaktionsmediums im Bereich von 5—7 in Kontakt bringt oder das Dihydrohalogenidsalz in a) (ii) als eine Suspension in Methylenchlorid mit einer wässrigen Basenlösung bei einer Temperatur im Bereich von —10 bis 50°C in Kontakt bringt.

7. Verfahren nach einem der Ansprüche 4—6, dadurch gekennzeichnet, dass man das Monohydrohalogenidbisimidatsalz, das in b) (ii) gebildet wurde, recyclisiert durch

a) Kontakt des Monohydrohalogenid-bisimidatsalzes mit einem Moläquivalent einer Base, unter Erzeugung eines Bisimidats, und

b) Kontakt des Bisimidats mit Chlorcyan unter Bildung eines N-Cyanoimidats.

8. Verfahren nach einem der Ansprüche 4 bis 7, dadurch gekennzeichnet, dass man das resultierende N-Cyanoimidat anschliessend bei einer Temperatur, die zum Ringschluss und zur Bildung einer Verbindung der Formel

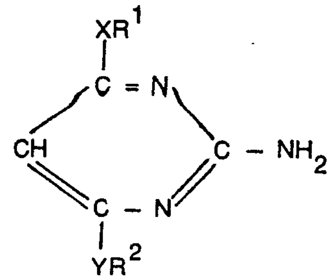

ausreicht, erwärmt.

9. Verfahren nach Anspruch 8, dadurch gekennzeichnet, dass man das N-Cyanoimidat a) unvermischt bei einer Temperatur über seinem Schmelzpunkt oder b) in einem inerten flüssigen Medium bei einer Temperatur im Bereich von 0 bis 200°C erwärmt, bis der Ringschluss vollständig ist.